# EUROPEAN PATENT SPECIFICATION

(11) **EP 2 217 203 B1**
(45) Date of publication and mention of the grant of the patent: **21.12.2011**
(21) Application number: 08846737.8
(22) Date of filing: 05.11.2008
(51) Int. Cl.: A61K 8/60, A61K 8/73, A61Q 19/10

(54) **PERSONAL CARE CLEANSING COMPOSITIONS COMPRISING HYDROXYPROPYL METHYL CELLULOSE AND ALKYL POLYGLUCOSIDES**
REINIGUNGSZUSAMMENSETZUNGEN ZUR KÖRPERPFLEGE MIT HYDROXYPROPYLMETHYLCELLULOSE UND ALKYLPOLYGLUCOSIDEN
COMPOSITIONS NETTOYANTES DE SOIN PERSONNEL COMPRENANT UNE HYDROXYPROPYL METHYL CELLULOSE ET DES ALKYLE POLYGLUCOSIDES

(30) Priority: 06.11.2007 US 985796 P
(43) Date of publication of application: 18.08.2010
(73) Proprietor: Dow Global Technologies LLC, Midland, MI 48674 (US)
(72) Inventor: SIMMONDS, Michael, CH-8805 Richterswil (CH); TAGLIAFERRI, David, CH-4314 Zeiningen (CH)
(74) Representative: Raynor, John
(86) International application number: PCT/US2008/082426
(87) International publication number: WO 2009/061779

(56) References cited:
- US-A- 5 166 333
- US-A1- 2002 173 434
- US-A1- 2003 223 950

## Description

### FIELD

The present invention relates to personal care compositions.

### BACKGROUND

Alkyl polyglycosides are potentially important nonionic surfactants for cosmetic applications. They are derived exclusively from renewable resources (sugar and vegetable oil) and are biodegradable, both aerobically and anaerobically. Furthermore, they are a less irritating form of surfactant, being recognized as having good compatibility with the eyes, skin, and mucous membranes. Alkyl polyglycosides are ideal for use in mild cleansing formulations or to improve the dermatological compatibility of certain formulations.

Despite these potential benefits, widespread use of alkyl polyglycoside surfactants in personal care formulations is prevented by their lack of solubility and difficulty in thickening. For example, currently available formulations require certain ethoxylates, like sodium laureth sulfate (SLES), to keep the alkyl polyglycoside surfactant in a single phase. This undermines the benefits of using alkyl polyglycoside surfactants, as ethoxylate surfactants can act as irritants, discouraging use in children's or sensitive skin products. Moreover, certain European markets have a negative bias towards many ethoxylated materials. Formulations including alkyl polyglycoside surfactants are known in the industry to be difficult to thicken, particularly when a betaine is present as a co-surfactant. However, commonly used thickeners in the industry which might offer a solution are also ethoxylated (e.g., PEGs), and thus less desirable for marketing in Europe.

Thus, what is needed are improved personal care formulations that include alkyl polyglycosides.

US 2002/173434 A1 (BEHLER ANSGAR [DE] ET AL) 21 November 2002 (2002-11-21) discloses (Claim 1) a cleaning composition comprising an alkyl polyglycoside surfactant. Additionaly, the composition can comprise a HPMC having a hydroxypropyl content of 1-15% and a methoxyl content of 15-30% (§ [0071]).

### SUMMARY

The present invention provides personal care compositions comprising hydroxypropyl methyl cellulose having a hydroxypropyl MS of 0.8 or greater and a methoxyl DS of 2 or greater, and a nonionic surfactant including an C8-C16 fatty alcohol alkyl polyglucoside, wherein the viscosity of the composition is greater than 1000 m Pa.s (1000 cps).

### DETAILED DESCRIPTION

"Personal care" refers to compositions that are to be topically applied to a person. In a preferred embodiment, the personal care composition is for cleansing, for example, facial cleansers, body cleansers, hair cleansers (shampoo), hand washes, and in some embodiments, toothpaste.

The term "MS" refers to average number of moles of hydroxypropyl groups attached by an ether linkage per mole of anhydroglucose unit. The term "DS" refers to the degree of methoxyl substitution per anhydroglucose unit. Hydroxypropyl methyl cellulose is generally available under the tradename METHOCEL (The Dow Chemical Company), and hydroxypropyl methyl cellulose having a hydroxypropyl MS of about 0.8 or greater and a methoxyl DS of about 2 or greater is available under the tradename METHOCEL 311 (The Dow Chemical Company).

The amount of methoxyl and hydroxypropyl groups in the claimed hydroxypropyl methyl cellulose can also be defined as a weight percentage. In one embodiment, the weight percent methoxyl corresponds to about 22-27% and the weight percent hydroxypropyl corresponds to about 25-32%.

One method of making a hydroxypropyl methyl cellulose having a hydroxypropyl MS of about 0.8 or greater and a methoxyl DS of about 2 or greater is disclosed in US Patent No. 4,614,545.

The hydroxypropyl methyl cellulose is preferably present in a range greater than about 0.2% by weight of the composition, preferably greater than about 0.25% by weight of the composition, and less than about 1.5% by weight of the composition, preferably less than about 0.95% by weight of the composition, more preferably less than about 0.65% by weight of the composition. Note that the weight percents are active concentrations, so for example, including water, 0.2% is 0.2 parts out of 100 parts total.

Similarly, in one embodiment, the hydroxypropyl methyl cellulose is present in a range from about 0.3% to about 0.6% by weight of the composition. In one embodiment, the hydroxypropyl methyl cellulose is present from about 0.3% to about 0.4% by weight of the composition. In one embodiment, the hydroxypropyl methyl cellulose is present from about 0.4% to about 0.5% by weight of the composition. In one embodiment, the hydroxypropyl methyl cellulose is present from about 0.5% to about 0.6% by weight of the composition. The recited ranges herein are given to amply illustrate certain features of the invention; however, additional ranges are understood to be contemplated.

the nonionic surfactant is a C8-C16 fatty alcohol alkyl polyglucoside. In one embodiments, the alkyl polyglucoside is cocoglucoside, such is available under the tradename PLANTACARE 818 UP (Cognis), having a C8-C16 alkyl portion. In one embodiment, the alkyl polyglucoside is capryl glucoside, such is available under the tradename PLANTACARE 810 UP (Cognis), having a C8-C16 alkyl portion. In one embodiment, the alkyl polyglucoside is lauryl glucoside, such is available under the tradename PLANTACARE 1200 UP (Cognis), having a C8-C16 alkyl portion. In one embodiment, the alkyl polyglucoside is decyl glucoside, such is available under the tradename PLANTACARE 2000 UP (Cognis), having a C8-C16 alkyl portion.

In one embodiment, the nonionic surfactant is present in a range from about 5% to about 25% by weight of the composition.

Optionally, the nonionic surfactant further includes a co-surfactant. In one embodiment, the co-surfactant is cocamidopropyl betaine as a co-surfactant. In a preferred embodiment, the nonionic surfactant includes a C8-C16 fatty alcohol glucoside and cocamidopropyl betaine. This surfactant mixture may present in a range from about 5% to about 25% by weight of the composition. In one embodiment, the surfactant mixture is present in a range from about 9% to about 15% by weight of the composition. In one embodiment, the surfactant mixture is present in a range from about 9% to about 10% by weight of the composition. In one embodiment, the surfactant mixture is present in a range from about 9.5% to about 24% by weight of the composition. In one embodiment, the surfactant mixture is present in a range from about 20% to about 25% by weight of the composition. Similarly, the invention includes the surfactant mixture present in a concentration greater than about 5% by weight of the composition, preferably greater than about 8.5% by weight of the composition, and less than about 25.5% by weight of the composition, preferably less than about 25% by weight of the composition.

In one embodiment, personal care compositions of the present invention are substantially free of ethoxylates. In one embodiment, "substantially free" means concentrations less than 0.1%, preferably less than 0.01%, or more preferably, less than 0.001%. In one embodiment, personal care compositions of the present invention are substantially free of sodium laureth sulfate in particular. As mentioned above, personal care compositions that contain alkyl polyglycoside but are substantially free of ethoxylates would be expected to exhibit multiple phases and problems in obtaining suitably thick consistencies. In contrast, in one embodiment, compositions of the present invention are in a single phase. Compositions of the present invention have a viscosity of the composition that is greater than 1000 cps.

In one embodiment, compositions of the present invention have a haze value of 10 or less, determined using a 20mm length quartz cell, or a haze value of 5 or less, determined using a 10mm length cell. The claimed hydroxypropyl methyl cellulose gives a high level of formulation clarity while simultaneously providing the caring type benefits (good formulation texture, foam feel, foam stability, improved combing and feel) desirable in cosmetic cleansing preparations.

In one embodiment, the present invention provides cleansing formulations for personal care that are substantially free of ethoxylates. In one embodiment, the cleansing formulation is a clear, single phase, formulation. In one embodiment, the cleansing formulation is a clear, single phase, formulation with a viscosity that is greater than 1000 cps.

Other optional cosmetically acceptable ingredients are contemplated for personal care compositions of the present invention. "Cosmetically acceptable" refers to ingredients typically used in personal care compositions, and is intended to underscore that materials that are toxic, irritating, or unpleasant smelling when present in the amounts typically found in personal care compositions are not contemplated as part of the present invention.
Examples of cosmetically acceptable ingredients for a cleansing agent include emollients, moisturizers, conditioners, beneficial agents, anti-aging agents, sunscreens, drug substances, skin protectants, thickeners, colorants, preservatives, pH adjustors, reducing agents, fragrances, foaming agents, boosters, flavors, astringents, antiseptics, insect repellants, anti-dandruff agents, strengtheners, preservatives, and biocides. In one embodiment, the cleansing agent comprises surfactant, water, and at least one cosmetically acceptable ingredient.

Emollients include oils or other fatty substances. The term "oil" means a fatty substance that is liquid at room temperature. Examples of oils include hydrocarbon-based oils of animal origin, such as squalene, hydrocarbon-based oils of plant origin, such as liquid triglycerides of fatty acids comprising from 4 to 10 carbon atoms, for instance heptanoic or octanoic acid triglycerides, or alternatively, oils of plant origin, for example sunflower oil, corn oil, soybean oil, marrow oil, grapeseed oil, sesame seed oil, hazelnut oil, apricot oil, macadamia oil, arara oil, coriander oil, castor oil, avocado oil, jojoba oil, shea butter oil, or caprylic/capric acid triglycerides, MIGLYOL 810, 812 and 818 (from Dynamit Nobel), synthetic esters and ethers, especially of fatty acids, for instance the oils of formulae R¹COOR² and R¹OR² in which R¹ represents a fatty acid residue comprising from 8 to 29 carbon atoms and R² represents a branched or unbranched hydrocarbon-based chain comprising from 3 to 30 carbon atoms, for instance purcellin oil, isononyl isononanoate, isopropyl myristate, 2-ethylhexyl palmitate, 2-octyldodecyl stearate, 2-octyldodecyl erucate or isostearyl isostearate, hydroxylated esters, for instance isostearyl lactate, octyl hydroxystearate, octyldodecyl hydroxystearate, diisostearyl malate, triisocetyl citrate and fatty alcohol heptanoates, octanoates and decanoates, polyol esters, for instance propylene glycol dioctanoate, neopentyl glycol diheptanoate and diethylene glycol diisononanoate, pentaerythritol esters, for instance pentaerythrityl tetraisostearate, lipophilic derivatives of amino acids, such as isopropyl lauroyl sarcosinate, such as is sold under the name ELDEW SL 205 (from Ajinomoto), linear or branched hydrocarbons of mineral or synthetic origin, such as mineral oils (mixtures of petroleum-derived hydrocarbon-based oils), volatile or non-volatile liquid paraffins, and derivatives thereof, petroleum jelly, polydecenes, isohexadecane, isododecane, hydrogenated isoparaffin (or polyisobutene), silicone oils, for instance volatile or non-volatile polymethylsiloxanes (PDMS) comprising a linear or cyclic silicone chain, which are liquid or pasty at room temperature, especially cyclopolydimethylsiloxanes (cyclomethicones) such as cyclopentasiloxane and cyclohexadimethylsiloxane, polydimethylsiloxanes comprising alkyl, alkoxy or phenyl groups, which are pendent or at the end of a silicone chain, these groups comprising from 2 to 24 carbon atoms, phenyl silicones, for instance phenyl trimethicones, phenyl dimethicones, phenyltrimethylsiloxydiphenylsiloxanes, diphenyl dimethicones, diphenylmethyldiphenyltrisiloxanes 2-phenylethyltrimethyl siloxysilicates and polymethylphenylsiloxanes, fluoro oils such as partially hydrocarbon-based and/or partially silicone-based fluoro oils, ethers such as dicaprylyl ether (CTFA name: dicaprylyl ether), and C₁₂-C₁₅ fatty alcohol benzoates (FINSOLV TN from Finetex), mixtures thereof.

Oils include mineral oil, lanolin oil, coconut oil and derivatives thereof, cocoa butter, olive oil, almond oil, macadamia nut oil, aloe extracts such as aloe vera lipoquinone, jojoba oils, safflower oil, corn oil, liquid lanolin, cottonseed oil, peanut oil, hydrogenated vegetable oil, squalane, castor oil, polybutene, sweet almond oil, avocado oil, calophyllum oil, ricin oil, vitamin E acetate, olive oil, silicone oils such as dimethylopolysiloxane and cyclomethicone, linolenic alcohol, oleyl alcohol, and the oil of cereal germs.

Other suitable emollients include, for example, dicaprylyl ether, C₁₂₋₁₅ alkyl benzoate, DC 200 FLUID silicone fluids (from Dow Coming Corp.), isopropyl palmitate, octyl palmitate, isopropyl myristate, hexadecyl stearate, butyl stearate, decyl oleate, acetyl glycerides, the octanoates and benzoates of C₁₂₋₁₅ alcohols, the octanoates and decanoates of alcohols and polyalcohols such as those of glycol and glyceryl, ricinoleates esters such as isopropyl adipate, hexyl laurate and octyl dodecanoate, dicaprylyl maleate, phenyltrimethicone, and aloe vera extract. Solid or semi-solid cosmetic emollients include glyceryl dilaurate, hydrogenated lanolin, hydroxylated lanolin, acetylated lanolin, petrolatum, isopropyl lanolate, butyl myristate, cetyl myristate, myristyl myristate, myristyl lactate, cetyl alcohol, isostearyl alcohol and isocetyl lanolate.

Moisturizers include 2-pyrrolidone-5-carboxylic acid and its salts and esters, alkyl glucose alkoxylates or their esters, fatty alcohols, fatty esters, glycols and, in particular, methyl glucose ethoxylates or propoxylates and their stearate esters, isopropyl myristate, lanolin or cetyl alcohols, aloe, silicones, propylene glycol, glycerol and sorbitol.

Conditioners include stearalkonium chloride, dicetyldimonium chloride, lauryl methyl gluceth-10 hydroxypropyldimonium chloride, and conditioning polymers such as polyquatemiuni-10, polyquaternium-24 and chitosan and derivatives thereof, including chitosan/pyrrolidone carboxylic acid mixtures and/or polyquaternium-24 hyaluronates. Examples of these include KYTAMER PC chitosan/pyrrolidone carboxylic acid mixture and/or BIOCARE HA-24 polyquaternium-24 hyaluronate, each available from The Dow Chemical Company.

Sunscreens include paraminobenzoic acid, avobenzone, cinoxate, dioxybenzone, homosalate, menthyl anthranilate, octocrylene, octyl methoxycinnamate, octyl salicylate, oxybenzone, padimate O, phenylbenzimidazole sulfonic acid, sulisobenzone, trolamine salicylate, titanium dioxide and zinc oxide, diethanotamine methoxycinnamate, digalloy trioleate, ethyl dihydroxypropyl PABA, glyceryl aminobenzoate, lawsone with dihydroxy acetone, and red petrolatum.

Examples of thickeners include at least one of carboxyvinyl polymers, such as the products sold under the names CARBOPOL and PEMULEN (INCI name: Acrylates/C₁₀₋30 alkyl acrylate crosspolymer; available from Noveon), polyacrylates & polymethacrylates, , such as the products sold under the names LUBRAJEL and NORGEL (from Guardian) or HISPAGEL (from Hispano Chimica), polyacrylamides, and sodium polyacrylate/dimethicone/cyclopentasiloxane/ tri-deceth-6/PEG-PPG-18/18 dimethicone, polyacrylamides, for example, polyacrylamide/C13-C14 isoparaffin/laureth-72-acrylamido-2-methylpropanesulfonic acid polymers and copolymers, poly(2-acrylamido-2-methylpropane-sulfonic acid) sold by Clariant (INCI name: ammonium polyacryldimethyltauramide), emulsified crosslinked anionic copolymers of acrylamide and AMPS, such as those sold under the name SEPIGEL 305 (INCI name: Polyacrylamide/C13-14 Isoparaffin/Laureth-7; from Seppic) and under the name SIMULGEL 600 (INCI name: Acrylamide/Sodium acryloyldimethyltaurate copolymer/Isohexadecane/Polysorbate 80; from Seppic), polysaccharide biopolymers, for instance xanthan gum, guar gum, carob gum, acacia gum, scleroglucans, chitin and chitosan derivatives, carrageenans, gellans, alginates, starches, associative polymers, associative polyurethanes, copolymers comprising at least two hydrocarbon-based lipophilic chains comprising from 6 to 30 carbon atoms, separated with a hydrophilic sequence, such as the polyurethanes sold under the names SERAD FX1010, SERAD FX1100 and SERAD FX1035 (from Hüls America), RHEOLATE 255, RHEOLATE 278 and RHEOLATE 244 (INCI name: Polyether-urea-polyurethane; from Rheox), DW 1206F, DW 1206J, DW 1206B, DW 1206G, and ACRYSOL RM 2020 (from Röhm & Haas), water-soluble vinyl polymer, and celluloses such as microcrystalline cellulose, carboxymethylcellulose, hydroxymethylcellulose and hydroxypropylcellulose (generally, a relatively higher molecular weight grade of cellulose, i.e., greater than about 80,000 (Mn), will find use primarily as a thickener).

Colorants include pigments, which are used especially in make-up, including metal oxide pigments, titanium dioxide, optionally surface-treated, zirconium oxide or cerium oxide, zinc oxide, iron oxide (black, yellow or red), chromium oxide, manganese violet, ultramarine blue, chromium hydrate and ferric blue, carbon black, pigments of barium, strontium, calcium or aluminum (for example D&C or FD&C), cochineal carmine, mica coated with titanium or with bismuth oxychloride, titanium mica with iron oxides, titanium mica with, especially, ferric blue or chromium oxide, titanium mica with an organic pigment, nacreous pigments based on bismuth oxychloride, goniochromatic pigments, for example pigments with a multilayer interference structure, reflective pigments, for example particles with a silver-coated glass substrate, glass substrate coated with nicket/chromium/molybdenum alloy, glass substrate coated with brown iron oxide, particles comprising a stack of at least two polymer layers, for instance MIRROR GLITTER (from 3M).

Dyes include water-soluble dyes such as copper sulfate, iron sulfate, water-soluble sulfopolyesters, rhodamines, natural dyes, for instance carotene and beetroot juice, methylene blue, caramel, the disodium salt of tartrazine and the disodium salt of fuschin, and mixtures thereof. Liposoluble dyes may also optionally be used.

Preservatives include alcohols, aldehydes, methylchloroisothiazolinone and methylisothiazolinone, p-hydroxybenzoates, and in particular methylparaben, propylparaben, glutaraldehyde and ethyl alcohol.

The pH adjustors, include inorganic and organic acids and bases and in particular aqueous ammonia, citric acid, phosphoric acid, acetic acid, and sodium hydroxide.

Reducing agents include ammonium thioglycolate, hydroquinone and sodium thioglycolate.

Fragrances include any component which provides a pleasant scent. Fragrances are generally aldehydes or ketones, and often oils obtained by extraction of natural substances or synthetically produced. Often, fragrances are accompanied by auxiliary materials, such as fixatives, extenders, stabilizers and solvents.

Biocides include antimicrobials, bactericides, fungicides, algaecides, mildicides, disinfectants, antiseptics, and insecticides.

The amount of optional ingredients effective for achieving the desired property provided by such ingredients can be readily determined by one skilled in the art.

### EXAMPLES

The following examples are for illustrative purposes only and are not intended to limit the scope of the present invention. All percentages are by weight of the active ingredient unless otherwise specified.

### Example 1

Exemplary personal care cleansing compositions contain the components recited in TABLES 1A-1B.

**TABLE 1A**

| | **Batch 1** | **Batch 2** | **Batch 3** | **Batch 4** | **Batch 5** | **Batch 6** |
|---|---|---|---|---|---|---|
| lauryl glucoside (50% solids; PLANTACARE 1200 UP; Cognis) | 5 | 7 | 9 | 3.6 | 5 | 7 |
| cocoglucoside (50% solids; PLANTACARE 818 UP; Cognis) | - - | - - | - - | 1.4 | 2 | - - |
| cocamidopropyl betaine (38% solids; TEGO F50; Degussa) | 15 | 15 | 15 | 15 | 15 | 15 |
| hydroxypropyl methyl cellulose (METHOCEL 311; The Dow Chemical Company) | 0.3 | 0.3 | 0.3 | 0.3 | 0.3 | 0.4 |
| Citric acid (* = amt sufficient to produce pH 5.7) | * | * | * | * | * | * |
| Water | Bal. | Bal. | Bal. | Bal. | Bal. | Bal. |

**TABLE 1B**

| | **Batch 7** | **Batch 8** | **Batch 9** | **Batch 10** | **Batch 11** | **Batch 12** |
|---|---|---|---|---|---|---|
| lauryl glucoside (50% solids; PLANTACARE 1200 UP; Cognis) | 4 | 4 | 4 | 2.5 | 4.5 | 4.5 |
| cocoglucoside (50% solids; PLANTACARE 818 UP; Cognis) | - - | - - | - - | - - | - - | - - |
| cocamidopropyl betaine (38% solids; TEGO F50; Degussa) | 5.7 | 5.7 | 5.7 | 5.7 | 5.7 | 7.6 |
| hydroxypropyl methyl cellulose (METHOCEL 311; The Dow Chemical Company) | 0.4 | 0.5 | 0.6 | 0.3 | 0.3 | 0.4 |
| Citric acid | * | * | * | * | * | * |
| Water | Bal. | Bal. | Bal. | Bal. | Bal. | Bal. |

| | | | | | | |
|---|---|---|---|---|---|---|
| * = amt sufficient to produce pH 5.7 | | | | | | |

For the purpose of illustration and clarification, the following representative example reflects the concentrations of ingredients used to prepare batch 7. A blend of APG (4 parts active) and CAPB (5.7 parts active) surfactants is prepared under mild agitation by heating the solid PLANTACARE 1200 UP (APG) to above 40°C and pouring it into a beaker containing the required weight of TEGO F50 (for other batches, if required by ingredient list, PLANTACARE 818 UP is then added). In a separate beaker, water is weighed and heated to 70°C in a bath. The hot water is removed from the bath and agitated with an overhead mixer at approximately 100 rpm. METHOCEL 311 powder (0.4 parts active) is immediately added over 1 minute. The polymer is left to hydrate under agitation while the water cools to room temperature over roughly 30 minutes. To the hydrated METHOCEL-containing solution is added the APG/CAPB mix. The mix is stirred for 10 minutes and the pH is adjusted to 5.7 with citric acid. The formulation is topped up to 100 parts with a small amount of additional water. The final formulation is single phased.

Batches 1-6 have a relatively high total surfactant concentration of greater than about 20wt%. Batches 7-12 have a relatively lower surfactant level of equal to or less than 12wt%.

### Example 2

Formulations made substantially according to the protocol of Example 1 representing Batches 1-12 were tested using a Brookfield viscometer DV-I (spindle 5, 20 rpm) at ~21°C. The viscosities are listed in TABLE 2 in cps.

**TABLE 2**

| **Batch** | **1** | **2** | **3** | **4** | **5** | **6** | **7** | **8** | **9** | **10** | **11** | **12** |
|---|---|---|---|---|---|---|---|---|---|---|---|---|
| Visc. | 200* | 920* | 2090 | 200* | 1000 | 1280 | 740* | 1040 | 1760 | 180* | 220* | 360* |

Batches where the viscosity is higher can be considered preferred embodiments of the present invention, but all exhibited a single phase that was stable for at least two weeks.

(examples not according to the invention)

### Example 3

Exemplary and comparative personal care cleansing compositions contain the components recited in TABLE 3.

**TABLE 3**

| | **Batch 13 (compar.)** | **Batch 14 (compar.)** | **Batch 15 (compar.)** | **Batch 16 (compar.)** | **Bat. 17** | **Bat. 18** |
|---|---|---|---|---|---|---|
| Hydroxypropyl methyl cellulose; METHOCEL 311 | - - | - - | 0.3 | 0.6 | 0.3 | 0.6 |
| Lauryl glucoside (52% actives; PLANTACARE 1200 UP) | 4 | 9 | - - | - - | 9 | 4 |
| Cocamidopropyl betaine (38% actives) | 5.7 | 15 | - - | - - | 15 | 5.7 |
| Citric acid | * | * | * | * | * | * |
| Water | Bal. | Bal. | Bal. | Bal. | Bal. | Bal. |

| | | | | | | |
|---|---|---|---|---|---|---|
| * = amt sufficient to produce pH 5.7 | | | | | | |

The hydroxypropyl methyl cellulose is added slowly to 70°C water under agitation, and allowed to hydrate for about 30 min.

The surfactants are combined at 40°C to form a mixture. The surfactant mixture is added to the hydrated hydroxypropyl methyl cellulose solution, and stirred for about 20 minutes. Citric acid (10% aqueous solution) is added to adjust the pH to about 5.7. The balance of the water is added.

### Example 4

Formulations made substantially according to the protocol of Example 3 representing Batches 13-18 were tested and their viscosity determined. The viscosities are listed in TABLE 4 in cps.

**TABLE 4**

| | **Batch 13 (compar.)** | **Batch 14 (compar.)** | **Batch 15 (compar.)** | **Batch 16 (compar.)** | **Batch 17** | **Batch 18** |
|---|---|---|---|---|---|---|
| Viscosity | 20 | 200 | 100 | 1120 | 1300 | 1980 |

All batches exhibited a single phase, and all but Batch 18 were clear.

### Example 5

Exemplary and comparative personal care cleansing compositions contain the following components 5 wt% lauryl glucoside (50% solids; PLANTACARE 1200 UP; Cognis), 2 wt% cocoglucoside (50% solids; PLANTACARE 818 UP; Cognis), 15 wt% cocamidopropyl betaine (38% solids; TEGO F50; Degussa), and 0.3 wt% of the polymers listed in TABLE 5.

**TABLE 5**

| **Polymer** | **MS** | **DS** | **Appearance** | **Phase** |
|---|---|---|---|---|
| Hydroxypropyl methyl cellulose; METHOCEL 311 | 0.8 | 2.0 | Clear | Single phase |
| Hydroxypropyl methyl cellulose; METHOCEL 40-100 (J type) (comparative) | 0.82 | 1.3 | Cloudy | Two phase |
| Hydroxypropyl methyl cellulose; METHOCEL 40-100 (J type) (comparative) | 0.82 | 1.3 | Cloudy | Single phase initially |
| Hydroxypropyl methyl cellulose; METHOCEL 40-202 (E type) (comparative) | 0.23 | 1.9 | Cloudy | Two phase |
| Methyl cellulose; METHOCEL A4M (comparative) | N/A | 1.8 | Cloudy | Two phase |
| Hydroxyethyl cellulose; CELLOSIZE QP 52000H (comparative) | N/A | N/A | Cloudy | Two phase |
| Quaternized hydroxyethyl cellulose; SOFTCAT SL-5 (comparative) | N/A | N/A | Cloudy | Two phase |

The polymers are hydrated to make aqueous solutions according to standard techniques, *i.e.*, cold water processable polymers were stirred into water at room temperature, METHOCEL 311 polymer and METHOCEL A4M polymer were added to water at about 70°C and allowed to cool slowly over 30 minutes as they are not cold water processable. The pH of each formulation is adjusted to 5.7. The surfactants are added.

As shown above, only the formulation within the claimed range of MS and DS can achieve a clear, single phase formulation. Haze (measure of clarity) was tested using a Nippon Denshoku COH-300a and a 20mm length cell. The haze value for the METHOCEL 311 hydroxypropyl methyl cellulose (having MS= 0.8, DS= 2.0) containing formulation was 5.36, whereas the haze value for the METHOCEL 40-202 hydroxypropyl methyl cellulose (having MS= 0.23, DS= 1.9) containing formulation was over 20. Visual perception of turbidity occurs around 10 on the haze scale when using a 20mm cell.

It is understood that the present invention is not limited to the embodiments specifically disclosed and exemplified herein. Various modifications of the invention will be apparent to those skilled in the art. Such changes and modifications may be made without departing from the scope of the appended claims.

Moreover, each recited range includes all combinations and subcombinations of ranges, as well as specific numerals contained therein.

## Claims

1. A personal care composition, comprising:
hydroxypropyl methyl cellulose having a hydroxypropyl MS where MS refers to the average mumber of moles of hydroxy propyl groups attached by an ether linkage per mole of an hydroglucose unit, of 0.8 or greater and a methoxyl DS, where DS refers to the degree of methoxy substitution per anhydroglucose unit, of 2 or greater; and
a C8-C16 fatty alcohol alkyl polyglucoside surfactant;
wherein the viscosity of the composition is greater than 1000 mPa.s (1000 cps).

2. The personal care composition of claim 1, wherein the composition is substantially ethoxylate-free.

3. The personal care composition of claim 1, wherein the composition is substantially free of sodium laureth sulfate.

4. The personal care composition of claim 1, wherein the composition has a haze value of 10 or less.

5. The personal care composition of claim 1, wherein the composition is in a single phase.

6. The personal care composition of claim 1, wherein the hydroxypropyl methyl cellulose is present in a range greater than 0.2% by weight of the composition and less than 1.5% by weight of the composition.

7. The personal care composition of claim 1, further comprising cocamidopropyl betaine as a co-surfactant.

8. The personal care composition of claim 7, wherein the surfactant mixture is present in a range from 9% to 15% by weight of the composition.

9. The personal care composition of claim 7, wherein the surfactant mixture is present in a range from 9% to 10% by weight of the composition.

10. The personal care composition of claim 7, wherein the surfactant mixture is present in a range from 9.5% to 24%by weight of the composition.

11. The personal care composition of claim 7, wherein the surfactant mixture is present in a range from 20% to 25% by weight of the composition.

12. The personal care composition of claim 7, wherein the hydroxypropyl methyl cellulose is present in a range from 0.3% to 0.6% by weight of the composition.

13. The personal care composition of claim 10, wherein the hydroxypropyl methyl cellulose is present in a range from 0.3% to 0.6% by weight of the composition.

14. The personal care composition of claim 11, wherein the hydroxypropyl methyl cellulose is present in a range from 0.3% to 0.4% by weight of the composition.

## Patentansprüche

1. Körperpflegezusammensetzung, umfassend:
Hydroxypropylmethylzellulose mit einer Hydroxypropyl-MS von 0,8 oder
größer, worin MS die mittlere Anzahl von Molen von Hydroxypropylgruppen, die durch eine Etherbindung gebunden sind, pro mol Anhydroglukoseeinheit bedeutet, und einer Methoxyl-DS von 2 oder größer, worin DS den Grad Methoxysubstitution pro Anhydroglukoseeinheit bedeutedt; und
ein C8-C16-Fettalkoholalkylpolyglukosid-oberflächenaktives Mittel; worin die Viskosität der Zusammensetzung größer als 1000 mPa·s (1000 cps)) ist.

2. Körperpflegezusammensetzung nach Anspruch 1, worin die Zusammensetzung im Wesentlichen frei von Ethoxylat ist.

3. Körperpflegezusammensetzung nach Anspruch 1, worin die Zusammensetzung im Wesentlichen frei von Natriumlaurylsulfat ist.

4. Körperpflegezusammensetzung nach Anspruch 1, worin die Zusammensetzung einen Trübungswert von 10 oder weniger hat.

5. Körperpflegezusammensetzung nach Anspruch 1, worin die Zusammensetzung einphasig ist.

6. Körperpflegezusammensetzung nach Anspruch 1, worin die Hydroxypropylmethylzellulose in einem Bereich von größer als 0,2 Gew.-% der Zusammensetzung und kleiner als 1,5 Gew.-% der Zusammensetzung vorliegt.

7. Körperpflegezusammensetzung nach Anspruch 1, weiterhin umfassend Cocamidopropylbetain als ein zusätzliches oberflächenaktives Mittel.

8. Körperpflegezusammensetzung nach Anspruch 7, worin das oberflächenaktive Gemisch in einem Bereich von 9 Gew.-% bis 15 Gew.-% der Zusammensetzung vorliegt.

9. Körperpflegezusammensetzung nach Anspruch 7, worin das oberflächenaktive Gemisch in einem Bereich von 9 Gew.-% bis 10 Gew.-% der Zusammensetzung vorliegt.

10. Körperpflegezusammensetzung nach Anspruch 7, worin das oberflächenaktive Gemisch in einem Bereich von 9,5 Gew.-% bis 24 Gew.-% der Zusammensetzung vorliegt.

11. Körperpflegezusammensetzung nach Anspruch 7, worin das oberflächenaktive Gemisch in einem Bereich von 20 Gew.-% bis 25 Gew.-% der Zusammensetzung vorliegt.

12. Körperpflegezusammensetzung nach Anspruch 7, worin die Hydroxypropylmethylzellulose in einem Bereich von 0,3.Gew.-% bis 0,6 Gew.-% der Zusammensetzung vorliegt.

13. Körperpflegezusammensetzung nach Anspruch 10, worin die Hydroxypropylmethylzellulose in einem Bereich von 0,3 Gew.-% bis 0,6 Gew.-% der Zusammensetzung vorliegt.

14. Körperpflegezusammensetzung nach Anspruch 11, worin die Hydroxypropylmethylzellulose in einem Bereich von 0,3 Gew.-% bis 0,4 Gew.-% der Zusammensetzung vorliegt.

## Revendications

1. Composition de soin personnel, comprenant :
- une hydroxypropyl-méthyl-cellulose dont l'indice d'hydroxypropyle MS ("MS" désigne le nombre moyen de moles de groupes hydroxy-propyle raccordés par chaînon de type éther, par mole de motifs d'anhydroglucose) vaut au moins 0,8 et dont l'indice de méthoxy DS ("DS" désigne le degré de substitution méthoxy par motif d'anhydroglucose) vaut au moins 2 ;
- et un tensioactif de type alkyl-polyglucoside d'alcool gras en C₈₋₁₆ ; laquelle composition présente une viscosité supérieure à 1000 mPa.s (1000 centipoises).

2. Composition de soin personnel conforme à la revendication 1, laquelle composition ne contient pratiquement pas de produit d'éthoxylation.

3. Composition de soin personnel conforme à la revendication 1, laquelle composition ne contient pratiquement pas de laureth-sulfate de sodium

4. Composition de soin personnel conforme à la revendication 1, laquelle composition présente un indice de trouble inférieur ou égal à 10.

5. Composition de soin personnel conforme à la revendication 1, laquelle composition se présente en une phase unique.

6. Composition de soin personnel conforme à la revendication 1, dans laquelle l'hydroxypropyl-méthyl-cellulose se trouve présente en une quantité représentant plus de 0,2 % du poids de la composition, mais moins de 1,5 % du poids de la composition.

7. Composition de soin personnel conforme à la revendication 1, qui comprend en outre de la coco-amido-propyl-bétaïne en tant que co-tensioactif.

8. Composition de soin personnel conforme à la revendication 7, dans laquelle le mélange de tensioactifs se trouve présent en une quantité représentant de 9 % à 15 % du poids de la composition.

9. Composition de soin personnel conforme à la revendication 7, dans laquelle le mélange de tensioactifs se trouve présent en une quantité représentant de 9 % à 10 % du poids de la composition.

10. Composition de soin personnel conforme à la revendication 7, dans laquelle le mélange de tensioactifs se trouve présent en une quantité représentant de 9,5 % à 24 % du poids de la composition.

11. Composition de soin personnel conforme à la revendication 7, dans laquelle le mélange de tensioactifs se trouve présent en une quantité représentant de 20 % à 25 % du poids de la composition.

12. Composition de soin personnel conforme à la revendication 7, dans laquelle l'hydroxypropyl-méthyl-cellulose se trouve présente en une quantité représentant de 0,3 % à 0,6 % du poids de la composition.

13. Composition de soin personnel conforme à la revendication 10, dans laquelle l'hydroxypropyl-méthyl-cellulose se trouve présente en une quantité représentant de 0,3 % à 0,6 % du poids de la composition.

14. Composition de soin personnel conforme à la revendication 11, dans laquelle l'hydroxypropyl-méthyl-cellulose se trouve présente en une quantité représentant de 0,3 % à 0,4 % du poids de la composition.
